# EUROPEAN PATENT SPECIFICATION

(11) **EP 0 871 001 B1**
(45) Date of publication and mention of the grant of the patent: **15.10.2003**
(21) Application number: 98112714.5
(22) Date of filing: 18.04.1994
(51) Int. Cl.: F25C 1/00

(54) **Method and apparatus for producing frozen particles using an entrapment zone of atomized cryogenic liquid droplets**
Verfahren und Vorrichtung zur Herstellung gefrorener Partikel mit einem Einfanggebiet für Tröpfchen einer zerstäubten Kryoflüssigkeit
Procédé et dispositif pour la production de particules congelées en utilisant une zone de piégeage de goutelettes de liquide cryogène atomisé

(30) Priority: 20.04.1993 US 49819
(43) Date of publication of application: 14.10.1998
(62) Divisional of application: 94105964.4
(73) Proprietor: DADE BEHRING INC., Deerfield, IL 60015-0778 (US)
(72) Inventor: Fermani, Nicholas Rocco, Newark, Delaware 19711 (US); Fletcher, Mark George, Newark, Delaware 19702 (US)
(74) Representative: Helbing, Jörg, Dr. Dipl.-Chem.

(56) References cited:
- EP-A- 0 284 837
- EP-A- 0 468 269
- EP-A- 0 478 118
- GB-A- 1 559 920
- NL-A- 6 814 641
- US-A- 3 788 095
- US-A- 4 952 224
- US-A- 5 196 049

## Description

### Field of the Invention

This invention relates to a method for producing frozen particles of a liquid and, more particularly, to a method having a liquid nozzle for introducing liquid droplets to be frozen and a plurality of flat spray cryogenic nozzles for creating an entrapment zone of atomized droplets of cryogenic fluid so as to freeze the liquid droplets to produce frozen particles.

### Background Art

The formation of physically uniform and chemically homogeneous spherical frozen particles from aqueous biological solutions and suspensions is essential to the manufacture of unit dose tablets for the pharmaceutical and diagnostic industries. Physically uniform particles minimize variability in bulk density and provide for tablet manufacturing processes that can produce tablets which vary in weight by no more than about one percent. Homogeneous chemical composition of the particles is needed to insure an accurate unit dose. From a manufacturing perspective it is desirable for frozen particles which are processed in various types of manufacturing tablet making equipment to exhibit the free flow properties characteristic of spherical particles. Furthermore, any manufacturing process for obtaining frozen particles with these desired characteristics should operate efficiently to offset the generally high cost of reagents and other raw materials.

Known techniques used in processing granular products include dry blending, direct compression, wet granulation, particle fabrication by coating product nuclei with discrete ingredients, fluidized bed granulation and spray freezing of frozen particles.

Spray freezing methods generally produce highly uniform particles as a result of their characteristically rapid freezing processes. However, spray freezing is generally an expensive technique due to the high cost of refrigerants and the relatively large product losses which can occur.

Typical methods of spray freezing include striking or impinging a solution to be frozen into or against a liquid refrigerant stream(s) or spray(s), spraying a solution to be frozen onto the surface of a bath of liquid refrigerant, and spraying solution to be frozen through a freezing zone. Examples of refrigerants typically used to accomplish this freezing include liquid nitrogen, liquid carbon dioxide, and fluorocarbons. Some of the disadvantages of these known methods are: insufficient throughput to support manufacturing sale needs, loss of product due to partial freezing and/or accumulation on the sides of the freeze chamber, and inefficient uses of cryogenic liquids.

DE-A-2 256 790 filed on November 20, 1972 and published June 6, 1974 discloses a process for deep freezing aqueous extracts with a freezing vessel having a conical lower section which provides for the impingement of a liquid product with a liquid refrigerant. Impingement occurs by one of three disclosed variations including downwardly spraying product and upwardly spraying refrigerants, upwardly spraying both product and liquid refrigerant, and both upwardly and downwardly spraying the product and upwardly spraying the liquid refrigerant. The disadvantage of this apparatus is the likelihood that frozen product will adhere to the liquid refrigerant nozzles and the funnel shaped bottom of the spray chamber, thus resulting in yield losses.

U.S. Patent No. 4,952,224, issued August 28, 1990, discloses an apparatus having a downwardly directed spray of atomized liquid fat and a radially inwardly directed plurality of individual spray jets of liquid nitrogen or carbon dioxide which surround and impinge against the downwardly directed spray of liquid fat droplets. The individual spray jets of this apparatus are likely to produce non-uniform agglomerations of the product due to the high velocity contact of the liquid refrigerant and the product. In addition, due to the limited time that the product is in contact with the liquid refrigerant, at least some product is unlikely to be frozen.

DE-A- 2,659,546 issued July 13, 1978, discloses an apparatus for freezing food products having an insulated freezing chamber with a conical lower section and a liquid refrigerant nozzle with a vertically downward directed spray located in the top center of the chamber. A plurality of product nozzles are distributed around and directed at the sides of the central refrigerant spray. Using this apparatus it is likely that unacceptably high particle size variations would occur during a continuous manufacturing process due to variations likely to occur in product supply and control systems, such as pressure changes in the product supply pump and pulsations within the nozzles.

EP-A-0478118 discloses an apparatus for producing frozen particles comprising:
an airtight adiabatic vessel having at a lower portion thereof a frozen particle outlet which can also serve as a gas outlet;
a material atomizer for spraying downward a material to be frozen in said vessel; and
a plurality of refrigerant atomizers arranged at regular intervals around an annular area surrounding the spray area of said material atomizer at upper portions inside of said vessel, each refrigerant atomizer being arranged to spray a refrigerant in a generally circumferential direction around the interior wall of the vessel.

Further, EP-A-0468269 discloses an apparatus utilizing a cryogenic liquid for producing metal powder from the molten metal, which apparatus comprises a cylindrical baffle surrounding the liquid metal tap hole of the apparatus.

There is needed an apparatus and process for producing uniformly, shaped, homogeneous frozen particles of a liquid, capable of supporting full scale manufacturing needs, which minimizes product yield losses due to accumulation of product on the inside of the freeze chamber and partially frozen particles.

### Summary of the Invention

Many of the disadvantages of the prior art are solved by the process of the present invention. The method of the present invention allow for the production of physically uniform and chemically homogeneous particles in a way that maximizes throughput or product yield and minimizes loss of product due to partial freezing and/or accumulation on the inner walls of the apparatus, and further minimizes the efficient use of cryogenic liquid. By physically uniform is meant of substantially the same size, shape and density.

The invention relates to a process for utilizing a cryogenic liquid for producing frozen particles of a liquid product in a housing as claimed in claim 1.

The process of the present invention offer significant advantages over the prior art by providing highly uniform, homogeneous frozen particles with minimum process yield losses. Yield losses are minimized during operation of the apparatus of the present invention in that the configuration of the cryogenic liquid nozzles form the sides of an entrapment zone of droplets of cryogenic liquid into which the liquid product droplets are introduced. The entrapment zone is created by introducing the cryogenic liquid into the housing in a downwardly directed planar spray pattern, using at least three cryogenic liquid nozzles. The liquid product droplets are contained within the entrapment zone, and directed in a downward direction such that the liquid product droplets do not impinge upon or break through the walls of the entrapment zone.

### Brief Description of the Figures

Figure 1 is a perspective view, partially cut away, of an upper portion of an apparatus constructed in accordance with a preferred embodiment of this invention for producing frozen particles of a liquid;
Figure 2 is a partially schematic representation of an apparatus for producing frozen particles using the apparatus of Fig. 1;
Figure 3 is a cross section of Figure 1 taken along the section lines 3-3 depicting the upper portion of the apparatus for producing frozen particles;
Figures 4a, 4b, and 4c are partially schematic representations of various views of a cryogenic liquid nozzle;
Figure 5 is another alternative embodiment of the invention depicting an ultrasonic nozzle which can be used as a liquid product nozzle in the apparatus of Fig. 1;
Figure 6 is a bottom view of the cryogenic liquid nozzles showing the flat planar spray pattern entrapment zone of this invention; and
Figure 7 is a schematic representation of the cryogenic liquid nozzle and product liquid nozzle spray patterns used in this invention.

### Description of the Preferred Embodiment of the Invention

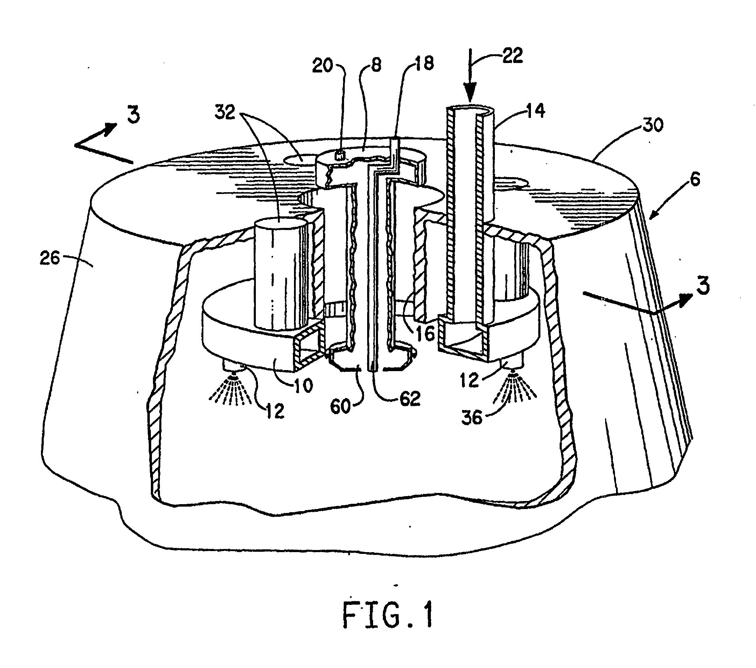

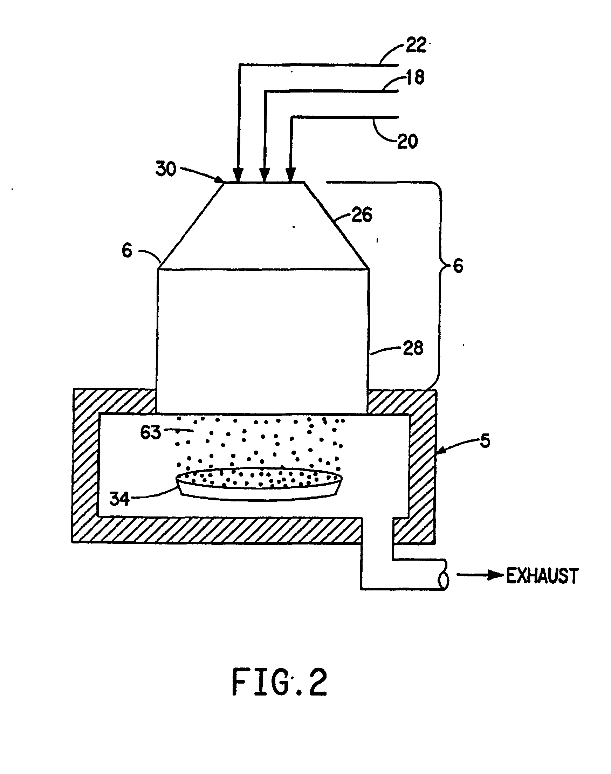

There may be seen in Figs. 1 and 2, an apparatus for producing frozen particles of a liquid product, the apparatus comprises a generally cylindrical housing 6, a liquid product nozzle, such as an atomization nozzle 8, for introducing droplets of the liquid to be frozen, and a plurality of cryogenic liquid nozzles 12 for introducing a cryogenic liquid into the housing 6 in a manner that directs the cryogenic liquid in a downward direction to form an entrapment zone or curtain cryogenic liquid droplets 36, whereby the cryogenic liquid freezes the liquid product droplets 63 to produce frozen particles.

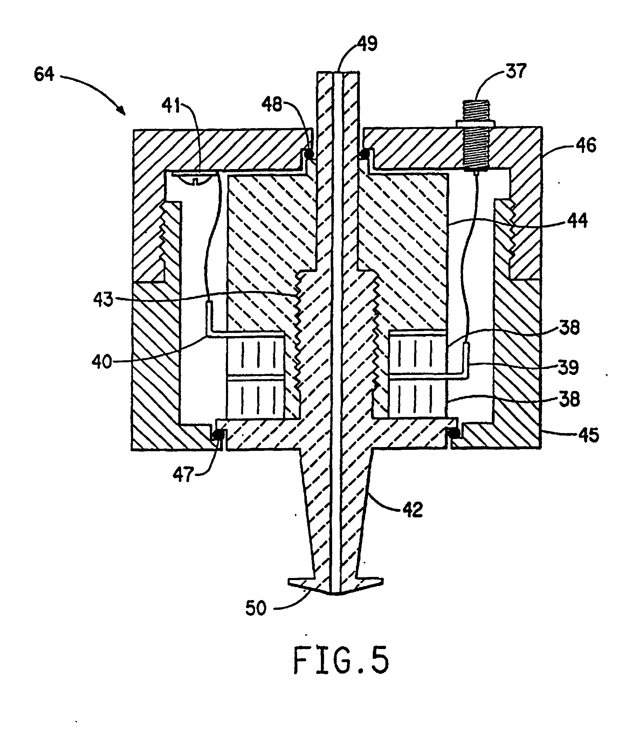

Figures 1 and 3 depict an atomization nozzle 8 suitable for use as the liquid product nozzle of the present invention. Such atomization nozzles 8 are known in the art and are available commercially. The atomization nozzle 8 is directed downwardly into the housing 6 so as to discharge liquid product in a vertically downward direction and designed so that a gas from a source not shown, but depicted by arrow 20, such as nitrogen or air, is forced under pressure through an annular channel 60, which surrounds a centrally located tube 62, through which the liquid product, shown by arrow 18, is discharged under pressure, such that the gas 20 breaks up the discharging liquid product into the liquid product droplets 63. Nitrogen is the preferred inert gas for use in the atomization nozzle 8. The end of the atomization nozzle 8 through which the liquid product is discharged is tapered inwardly providing a thin annulus through which the gas escapes at high velocity disposed about the liquid product. Such atomization nozzles 8 are generally useful for producing droplets in the size range of about 8 to about 4300µm.

Several examples of known types of droplet forming nozzles or atomization nozzles can be used as the liquid product nozzle, including ultrasonic nozzles, hydraulic nozzles, and pneumatic nozzles. An example of a suitable ultrasonic nozzle for use in the present invention is the Model 8700 nozzle available from Sono-Tek Corp., Poughkeepsie, N.Y. and an example of a suitable hydraulic nozzle is Model SU-5 available from Spraying Systems Co. (Wheaton, Ill.). Pneumatic nozzles are preferred.

Referring to Figures 1 and 2, the housing 6 has an upper plate 30 and a vertically disposed cylinder 28 having a diameter larger than the diameter of the upper plate 30, the upper plate 30 and cylinder 28 connected by a truncated conical section 26 having an upper conical diameter the same as the diameter of the upper plate 30 and a lower conical diameter the same as the lower diameter of the cylinder 28.

The diameter of the upper plate 30 should be sufficiently large to allow for the presence of a plurality of cryogenic liquid nozzles 12, the liquid product nozzle, such as an atomization nozzle 8, and a baffle 16 (if used) between the plurality of cryogenic liquid nozzles 12 and the liquid product nozzle.

The truncated conical section 26 should preferably be designed so that it is of sufficient length to allow for the formation of the entrapment zone of cryogenic liquid droplets.

The lower diameter of the cylinder 28 is defined by the lower conical diameter of the truncated conical section 26 and the cylinder should be of sufficient length to enclose and direct the frozen product particles and any gaseous cryogen which may have evaporated from the cryogenic liquid into the lower end of the cylinder 28 of the housing 6.

Preferably the apparatus is provided with a collection means 34 for the frozen particles. Any collection means 34 large enough to capture and hold the frozen particles produced in the housing 6 can be used including relatively simple devices such as trays which are manually operated as well as fully automated conveyor type systems for transporting the frozen particles away from the apparatus of the invention. The collection means 34 should be kept at a temperature below the freezing point of the frozen particles to insure that the frozen particles do not melt; such a temperature can be maintained, preferably, using the cooling effect resulting from the evaporation of cryogenic liquid to gaseous cryogen.

A baffle 16 is included between the cryogenic liquid nozzles 12 and the atomization nozzle 8. Such a baffle 16 serves to shield the liquid product nozzle 8 from the cryogenic liquid introduced into the housing 6 by the cryogenic liquid nozzles 12. The baffle 16 can be supported by any means sufficient to hold it between the atomization 8 and the cryogenic liquid nozzles 12 and should extend between the atomization nozzle 8 and the liquid cryogen nozzles 12 to a depth sufficient to shield the product nozzle 8.

By "liquid product" is meant any liquid which is to be frozen into uniform spherical particles. The preferred liquid products are aqueous solutions useful as diagnostic reagents or pharmaceutical reagents, including but not limited to solutions containing dissolved proteins such as enzymes, antibodies, antigens, vitamins, and hormones, solutions of other biological materials such as nucleic acids, antibiotics, and various drugs. Examples of such diagnostic reagents include aqueous solutions of biologically active substances such as nicotinamide adenine dinucleotide (NAD), which can be used in toxicology testing for analytically determining lactic acid and ethyl alcohol, and NAD-reduced disodium salt trihydrate (NADH), which can be used in analytically determining alpha-hydroxybutyrate dehydrogenase, the amount of which in turn can be related to the amount of the isoenzymes LD1 and LD2 of lactate dehydrogenase (LDH). These biologically active substances are preferably combined with an excipient such as mannitol or trehalose, and a lubricant such as carbowax. Other examples of suitable liquid products for use as diagnostic reagents and which can be frozen in accordance with the present invention include solutions of indicator compounds such as the organic dye dichloroindophenol, useful in determining pseudocholinesterase (PCHE), and liquid suspensions such as for example, a slurry of creatine kinase MB (CKMB) monoclonal antibody-coated chromium dioxide particles suspended in water, which can be used to test for the MB isoenzymes of creatine kinase.

One of the important aspects of the present invention is that frozen particles produced using the apparatus and process of this invention retain the biological and chemical characteristics and properties of the liquid product from which they are produced. For example, liquid products containing dissolved proteins such as enzymes can be frozen using the present invention to produce particles which retain the enzymatic activity of the aqueous liquid product.

By "liquid product nozzle" is meant any nozzle which can be used to introduce droplets of liquid product to be frozen into the apparatus of the present invention. The droplets of liquid product can vary depending on the desired frozen particle size. The liquid product nozzle therefore should be chosen so as to provide for droplets of a size sufficient to provide for the desired frozen particles. The preferred particle size range for the frozen particles produced using the apparatus of the present invention is about 75 to 600 µm.

Several types of cryogenic liquid nozzles are suitable for use with the present invention. A cryogenic liquid nozzle which produces a planar such as a flat or curved spray pattern is preferred. An example of a suitable nozzle for use as a cryogenic liquid nozzle is Model #HVV 9510, available from Spraying Systems, Inc.

One of the important features of the present invention is that the cryogenic liquid nozzles 12 are positioned so that they discharge cryogenic liquid to produce a spray pattern which forms an entrapment zone or a curtain around the liquid product droplets 63. The entrapment zone is defined by an interior region formed by a substantially continuous, annular downwardly directed circumferential wall of cryogenic liquid. At least 3 liquid nozzles are required to form the entrapment zone. The entrapment zone formed by 3 cryogenic liquid nozzles is in the shape of a triangle.

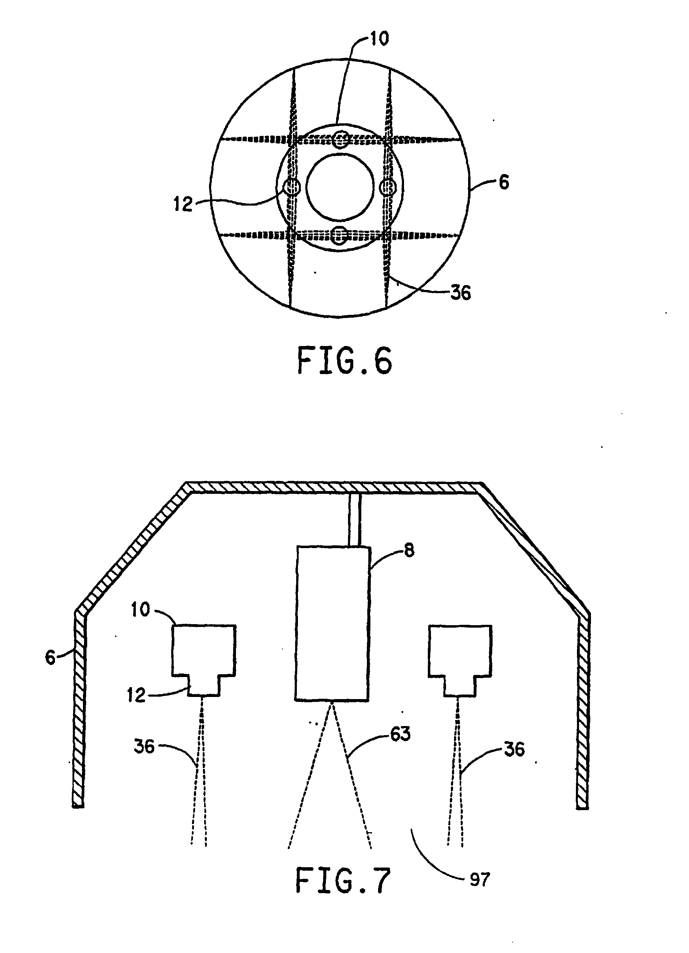

Preferably 4 to 6 cryogenic liquid nozzles are used, each nozzle producing a downwardly directed planar spray pattern. The entrapment zone produced using such a preferred spray pattern with four such nozzles is in the shape of a box such as a square or rectangle. Figure 6 depicts such a configuration.

By forming an entrapment zone around the liquid product droplets 63 the apparatus of the present invention provides for a continuous high yield manufacturing process which can produce frozen particles of a uniform spherical size and homogeneous chemical composition which do not impinge against or adhere to the sides of the apparatus housing 6. The cryogenic liquid nozzle 12 has a slit shaped orifice which (Figure 4a), is available with different slit sizes to discharge the cryogenic liquid as a flat or planar spray pattern with the planar portion defining various spray angles.

Figure 7 illustrates the entrapment zone 97 around the spray solution 63 formed by the cryogenic liquid spray 36.

By "housing" is meant a chamber in which the liquid product droplets are frozen. The housing 6 confines any gaseous cryogen which may have evaporated from the cryogenic liquid. The housing 6 should be vented to allow for the escape of any evaporated gaseous cryogen. A suitable housing 6 for use with the present invention can have a variety of shapes including rectangular, square, and cylindrical shapes. An upper portion of the housing 6 is preferably used for the introduction of the cryogenic liquid and the liquid product droplets 63, and for the formation of the frozen particles. A lower portion of the housing 6 is preferably used for collecting the frozen particles and venting any gaseous cryogen which may have evaporated from the cryogenic liquid. The housing 6 need not be insulated; an uninsulated housing 6 is preferred.

The preferred configuration of the cryogenic liquid nozzles 12 is a plurality of four nozzles positioned in a ring manifold 10. The preferred cryogenic liquid nozzles 12 produce a downwardly directed flat or planar spray pattern. The cryogenic liquid is introduced into the ring manifold 10, shown by 22, through a tube 14. The use of a plurality of nozzles screwed into a manifold for the cryogenic liquid nozzles 12 provides for the introduction of flat streams of cryogenic liquid shown by 36 into the housing 6. These flat sprays of cryogenic liquid should be sufficient to produce the maximum amount of frozen particles for a given amount of liquid product and should provide for a uniform environment of liquid cryogenic droplets and gaseous cryogen within the housing 6 of the apparatus in order to be useful for the continuous manufacturing of frozen particles. The cryogenic liquid spray droplets should be sufficiently small so that cryogenic liquid does not run out the bottom of the housing 6. The formation of an entrapment zone produced by a plurality of cryogenic liquid flat spray, is important for providing for the optimal heat transfer between the cryogenic liquid and the liquid product droplets 63.

Preferably, the temperature at the lower cylinder 28 outlet is maintained at about -100° to about -180° C. This temperature range can be obtained typically by choice of cryogenic liquid, adjustment of cryogenic liquid flow rate, and the sizes and quantity of nozzles. If this temperature range cannot be achieved, the liquid product flow rates can be adjusted so that the liquid product flow rate into the apparatus can be lowered.

Initially the liquid product flow rate can be adjusted to about 300 milliliters per minute (ml/min) and the atomization gas pressure can be adjusted to about 11 pounds per square inch (psi). By adjusting liquid product flow rate and atomization pressure a desired particle size can be achieved. For example, an undesirably small particle size can be corrected by lowering the atomization pressure. Other variables which can be optimized to achieve a desired particle size include cryogenic liquid flow rate, choice of type of liquid product and cryogenic liquid nozzles 12, and choice of housing 6.

Figure 5 depicts an ultrasonic nozzle assembly 64 suitable for use as the liquid product nozzle of the present invention. Such ultrasonic nozzle assemblies 64 are known in the art and are commercially available. Ultrasonic nozzles produce droplets of the liquid product by electrically generated vibrations of the nozzle and are typically useful for producing droplets in the size range of about 10 to about 100 µm. The ultrasonic nozzle assembly 64 has a nozzle 42 with an axially projecting threaded boss 43 and a hollow core 49. A pair of piezoelectric crystals 38, each having an associated electrode 39,40 are received on the boss 43. The crystals 38 are held in place by threading the back piece 44 onto the boss 43. The nozzle 42, crystals 38, electrodes 39,40 and back piece assembly 44 is elastically supported in the threadably engaging front housing 45 and rear housing 46 by the front and rear O-rings,47 and 48 respectively. An ultra high frequency electrical signal is supplied to the piezoelectric crystal discs 38,39 through the connector 37 to the input electrode 39, with a ground being provided through electrode 40 to the ground lug 41. The electrical signal causes the crystals 38,39 to expand and contract at the electrical excitation frequency, thus causing the nozzle 42 and back piece 44 to vibrate by virtue of their elastic mounting on O-rings 47 and 48. Liquid product that is introduced into the hollow core 49 absorbs some of the vibrational energy. The vibrational energy sets up wave motions in the liquid whose peaks become unstable and break away from the liquid mass beneath, causing a fine mist at the tip of the nozzle 50.

By "cryogenic liquid" is meant any liquid capable of freezing the liquid spray of reagent into particles under atmospheric conditions. Various cryogenic liquids which can be used in the present invention include nitrogen, carbon dioxide, argon and various fluorocarbons. Preferably the cryogenic liquid used is nitrogen.

By "cryogenic liquid nozzle" is meant any nozzle for introducing a cryogenic liquid into the apparatus of the present invention.

### Example 1

### Construction of an Apparatus for Producing Frozen Particles of a Liquid Product

An example of an apparatus designed and built for producing frozen particles of a liquid product in accordance with the present invention consisted of two circular sections and a top plate 30 of Figure 1 welded to each other.

The lower section was a 35.6 cm (14 inch) diameter, 122 cm (48 inch) high open-ended vertically disposed lower cylinder Figure 2 (28). The truncated conical middle section, Figure 2 (26), had a lower diameter of 35.6 cm (14 inch), an upper diameter of 24.1 cm (9½ inch), and a height of 25.4 cm (10 inch). The top plate, Figure 1 (30), consisted of 24.1 cm (9½ inch) diameter disk with a 7.6 cm (3 inch) diameter hole in its center and a 3.8 cm (1.5 inch) baffle, Figures 1 and 3 (16), extending downward.

All of the sections including the top plate 30 of the vessel were constructed using 0.318 cm (0.125 inch) thick, 304 grade stainless steel and the entire vessel was mounted thru the top of a product collection box (5).

The cryogenic liquid nozzles, Figure 1, (12), were nozzles screwed into a hollow ring manifold, Figure 1 (10), with an inside diameter of 8.9 cm (3½ inch), an outside diameter of 15.2 cm (6 inch), and a 2.5 cm (1 inch) by 1.9 cm (¾ inch) rectangular shaped cross section using 0.475 cm (0.187 inch) thick 304 stainless steel.
The cryogenic liquid nozzles (12) were 4 Spray Systems Model 9510, equally spaced around the bottom of a ring manifold, Figure 1 (10). The nozzles 12 were disposed about the atomization nozzle 8.

The cryogenic liquid was introduced into the housing 6 of the apparatus by forcing it through the hollow interior of the ring manifold 10 and out of the nozzles Figure 1 (36).

Three equally spaced 2.5 cm (1 inch) long, 1.9 cm (¾ inch) diameter vertical posts, Figure 1 (32), were attached to the top of the ring manifold 10 to support it within the housing 6. The bottom portion of the posts were welded to the ring. The top portion of the posts were drilled and tapped for 0.64-50.8 × 1.9 cm (¼-20 × ¾ inch) screws which provided a means for centrally mounting the ring manifold 10 to the top plate 30 of the vessel. A conventional bulk head fitting (not shown) was also attached to the top of the ring manifold 10 which allowed a one-half inch diameter cryogenic liquid feed line, Figures 1 and 3 (14), to be attached to and supply cryogenic liquid, Figures 1, 2, and 3 (22), to the hollow interior of the ring manifold 10.

The liquid product nozzle was a pneumatic atomization nozzle Figure 1 (8), with a 1.1 millimeter (mm) opening, (Model SU-5, Spraying Systems, Inc.). The atomization nozzle 8 was centrally mounted using a cross-bar in the central hole of the upper plate 30 such that the tip of the atomization nozzle 8 was even with the tips of the cryogenic liquid nozzles (12). A peristaltic pump (pump model no. 7520-00, Master Flex Co.) was used to feed the liquid product to the atomization nozzle 8.

### Example 2

### Producing Frozen Particles of A CKMB Diagnostic Reagent Useful in a Creatine Kinase-MB (CKMB) Immunoassay

Into a 10 liter (L) stainless steel pot was added 1703 milliliters (ml) of deionized water. The following components were added, mixed, and allowed to dissolve in order: 360 trehalose (Sigma AF, St. Louis, Missouri), 95 polyethylene glycol (PEG 8000) (Sigma AF, St. Louis, Missouri), 215 bovine albumin (Miles Inc., Kankakee, Il.), 252 sodium chloride (VWR Scientific, Bridgeport, NJ.), 1.8g magnesium chloride (VWR Scientific, Bridgeport, NJ.), 254 disodium PIPES buffer, (Research Organics, Inc., Cleveland, OH.), 39 PIPES (Research Organics Inc., Cleveland, OH.), 125 ml of a CKMB conjugate solution consisting of 1:1 ratio of F(ab')2 anti-CKMB antibody fragments and β-galactosidase (prepared as described below), and 1.1g mouse IgG antibody (Scantibodies Laboratory, Santtee, CA.) to eliminate non specific binding.

The cell lines producing the monoclonal antibodies employed were obtained using the procedure described in U.S. Patent No. 4,912,033 and in Vaidya et al., Clin. Chem. 32(4): 657-663 (1986), the disclosures of which are hereby incorporated by reference.

The anti-CKMB monoclonal antibodies so obtained were purified and isolated using affinity chromatography on Protein A Sepharose (Pharmacia Fine Chemicals, Uppsala, Sweden). Protein A is a polypeptide (MW 42,000) isolated from Staphylococcus aureus which binds immunoglobin without interacting with the antigen binding site.

While the above described method is preferred, monoclonal antibodies can be purified using any number of standard techniques such as ammonium sulfate precipitation dialysis, affinity chromatography, ion exchange chromatography, etc. These and other methods for the isolation and purification of monoclonal antibodies are described in general by Goding, Monoclonal Antibodies: Principles and Practice, Academic press, London and New York, 1983 and in U.S. Patent 4,533,496.

The anti-CKMB monoclonal antibody used to produce the immunoreactive fragment described below was obtained as described above. The clone number was 2580 CC 4.2, and the monoclonal antibody was an IgG2b subclass.

The purified anti-CKMB monoclonal antibody was dialyzed overnight at 4 degrees C (°C) against an acetate buffer containing 100 millimolar (mM) sodium acetate and 150 mM sodium chloride, pH. 3.5. The dialyzed antibody solution was diluted to a concentration of 5 milligrams per milliliter (mg/ml) using the acetate buffer. The antibody solution was placed in a water bath at 37°C for about 5 to 10 minutes.

A 10 mg/ml solution of pepsin (Sigma Chemical Co., St. Louis, MO) was prepared in the acetate buffer. The amount of pepsin required to give a weight ratio of antibody to pepsin of 50:1 was determined and the determined amount of pepsin solution was added to the antibody solution as the antibody solution was stirred. The mixture was incubated for about 10-15 minutes. The reaction was then stopped by slowly adding 3.5 molar (M) Tris base drop wise until the pH of the solution was in the range of 7.0 to 8.0. The resulting F(ab')2 preparation was then passed through 15-20 ml of Sepharose having Protein A bound to it in a 2.2 × 25 centimeter (cm) column at a flow rate of about 4-4.5 ml per hour. The protein peak was monitored by recording the absorbance of the fractions at 280 nm. The protein peak was collected and concentrated to about 30mg/ml using an Amicon stirred cell fitted with a 62 mm PM 30 membrane filter (both purchased from the Amicon Corp.). The sterilized F(ab'2) concentrate was filtered and stored at -20°C.

The sterilized F(ab')2 concentrate was coupled to β-galactosidase conjugate using the procedure substantially as described by Kitagawa et al., Enzyme labeling with N-hydroxysuccinimidyl ester of maleimide in "Enzyme Immunoassays, Ishikawa et al., Eds., pp 81-90 (1981). Anti-CKMB monoclonal antibody F(ab')2 fragment was dialyzed against an antibody dialysis buffer containing 20 mM phosphate buffer, 300 mM NaCl, pH 7.0). One mole of F(ab')2 was mixed with 30 moles of N-succinimidyl, 4 (N-maleimido methyl) cyclohexane-1-carboxylate (SMCC) and incubated at room temperature for 35 min with constant stirring. The mixture was loaded on a Sephadex G-25 column (2.2 × 13 cm) equipped with the UV detector (absorbance 280 nm). The activated F(ab.')2 fragment was eluted using the antibody dialysis buffer. The protein peak was collected, its volume recorded and the protein concentration estimated. One mole of E. coli β-galactosidase (Boehringer Mannheim) equivalent to 1 mole of SMCC activated F(ab')2 was dissolved in the antibody dialysis buffer. Activated F(ab')2 was mixed with β-galactosidase and incubated for at least 25 minutes at 25°C with constant stirring. Synthesis of the conjugate was monitored using an HPLC (LKB) equipped with a 100 µl loop GF 450 analytical column. The reaction was quenched when the leading peak extended beyond the second peak on the chromatogram by adding 10 µl of 0.1 M N-ethylmaleimide solution for every ml of conjugate reaction mixture. The mixture was concentrated to 4.0 ml using an Amicon stir cell and YM 100 filter (both purchased from the Amicon Corp.). Conjugate concentrate was filtered through 0.2 µ syringe filter and purified using LKB HPLC equipped with 1 ml loop GF 450 column, UV monitor, fraction collector and chart recorder. Appropriate fractions were collected and pooled and absorbance was measured at a wavelength of 280 nm to estimate the protein concentration. The resulting concentrate was filtered, sterilized and stored at 40°C. Conjugate concentrate was diluted as needed in a β-galactosidase conjugate dilution buffer (33.5 g PIPES (piperazine-N, N'-bis (2-ethanesulfonic acid)) 0.2 g MgCl₂, 29.2 g NaCl, 100 g bovine serum albumin, and 0.167 g mouse IgG per liter of deionized water, pH 7.0) for the CKMB assay.

The solution was adjusted to a final volume of 4.8L with deionized water and filtered through a 5 µm filter (Gelman Sciences, Annarbor Mich. (product No. T 505141)

Liquid nitrogen from a 2715 kg [60,000 pound (1b.)] tank was fed through a 1,27cm (one-half inch)diameter flexible steel tube at flow rate of 3.2 kilograms per minute (Kg/min) to the ring manifold having the cryogenic liquid nozzles (12). Liquid nitrogen was thereby forced out of the cryogenic liquid nozzles. A steady state temperature measured centrally at the extreme bottom of the housing 6 was in the range of -150 to -170 degrees C (°C). An 116 cm² (18 inch square) 5.1 cm (2.0 inch) deep collection tray FIG (2) 34 was pre-chilled to -35°C and placed about 5.1 cm (2 inch) below the opening of the vessel.

Nitrogen gas at a pressure of 0.69 bar (10 pounds per square inch (psi)), Figure 1, 2, and 3 (20), and at a flow rate of 300 milliliters per minute (ml/min), was fed to the atomization nozzle.

Frozen particles were collected in the collection tray 34 and manually distributed in the tray 34 to form a uniformly thick layer of frozen liquid product particles about 1.59 cm (% inch) deep. The liquid product and nitrogen gas flows to the atomization nozzle were stopped and the production run of frozen particles was completed.

The average particle size was determined to be 402 µm by filtering the particles through a series of known size sieves, with the size distribution shown in Table 1.

**TABLE 1**

| Size (microns) | % of total particles |
|---|---|
| >590 | 16.4 |
| 505-590 | 19.6 |
| 335-504 | 25.2 |
| 215-334 | 10.8 |
| 165-215 | 7.6 |
| 110-164 | 14.0 |
| 75-109 | 6.4 |

β-galactosidase activity of the above liquid product was measured both before and after spray freezing using the aca® discrete clinical analyzer (E. I. duPont de Nemours and Company, Wilmington, DE 19898). The pre spray freezing sample was obtained by diluting 1 mL of the liquid product with 20 mL of water. A post freezing sample was obtained by dissolving 1 gm of the frozen liquid product in 4 mL of water and diluting this solution with 80 mL of water. The prepared samples were placed in standard aca sample cups. The sample cups along with 3 aca MCKMB packs (standard aca® discrete clinical analyzer packs available from E. I. Du Pont de Nemours and Company, Wilmington, De) were loaded on the aca. The aca dispensed 100 µl of sample, 2mL of phosphate buffer, pH 7.8, and 2.9 mL of water into each MCKMB pack. Breaker mixer 1, a component of the aca® discrete clinical analyzer which breaks tablet reagents in the pack and facilitates the mixing of the reagents, was utilized to dissolve and mix the pack reagents with the sample. Bound enzyme reacted with chlorophenol red galactoside (CPRG) contained in the pack reagents at 37°C to form chlorophenol red (CPR). After 4.2 minutes the absorbance of the contents of the pack was measured at 577 and 600 nm wavelengths. 577 was the primary wavelength at which the CPR has maximum absorbance, and 600nm was the blanking wavelength. The 600 nm reading was subtracted from the 577 reading to eliminate interference due to suspended particles and the resulting average absorbance number was multiplied by 20 due to the 1 to 20 dilution of the sample. The results, indicated minimal loss of enzymatic activity due to spray freezing. Thus, the usefulness of the present invention in producing frozen particles of diagnostic reagents which retain their biological activity upon freezing has been demonstrated.

## Claims

1. A process for utilizing a cryogenic liquid for producing frozen particles of a liquid product in a housing (6), the process comprises:
(a) producing a substantially continuous, annular downwardly directed circumferential wall of cryogenic liquid (36) using at least three cryogenic liquid nozzles (12) to define an interior entrapment zone (97); and
(b) introducing droplets of the liquid product (63) into the entrapment zone (97) using a liquid product nozzle (8) positioned in the housing (6),
whereby the cryogenic liquid (36) freezes the liquid product droplets (63) to produce frozen particles, **characterized by**
(c) providing said housing with a baffle (16) positioned above an outlet tube (62) within the liquid product nozzle (8) to surround a sufficient portion of the liquid product nozzle (8) to shield the liquid product nozzle (8) from the cryogenic nozzles (12) whereby to prevent freezing of the liquid product within the liquid product nozzle (8).

2. The process of claim 1 wherein the liquid product is an aqueous solution.

3. The process of claim 2 wherein the aqueous solution contains proteins.

4. The process of claim 1 wherein the liquid product particles have a diameter of about 75 to 590 microns.

5. The process of claim 1 wherein the liquid product particles are spherical.

6. The process of claim 1 wherein the cryogenic liquid is applied by disposing the cryogenic liquid circumferentially about the liquid product droplets.

## Patentansprüche

1. Verfahren zur Verwendung einer kryogenen Flüssigkeit zur Erzeugung von gefrorenen Teilchen aus einem flüssigen Produkt in einem Gehäuse (6), wobei das Verfahren folgendes umfasst:
(a) Herstellen einer im Wesentlichen kontinuierlichen, ringförmigen, nach unten gerichteten umlaufenden Wand aus kryogener Flüssigkeit (36) unter Verwendung von wenigstens drei Düsen für die kryogene Flüssigkeit (12), so dass eine innere Einschlusszone (97) definiert wird; und
(b) Einführen von Tröpfchen des flüssigen Produkts (63) in die Einschlusszone (97) unter Verwendung einer Düse für das flüssige Produkt (8), die sich in dem Gehäuse (6) befindet;
wobei die kryogene Flüssigkeit (36) die Tröpfchen des flüssigen Produkts (63) gefrieren lässt, wobei gefrorene Teilchen entstehen, **dadurch gekennzeichnet, dass**
(c) das Gehäuse mit einer Prallwand (16) versehen ist, die sich oberhalb eines Ausgangsrohrs (62) innerhalb der Düse für das flüssige Produkt (8) befindet, so dass sie einen ausreichenden Teil der Düse für das flüssige Produkt (8) umgibt, um die Düse für das flüssige Produkt (8) gegenüber den Düsen für die kryogene Flüssigkeit (12) abzuschirmen, wodurch ein Gefrieren des flüssigen Produkts innerhalb der Düse für das flüssige Produkt (8) verhindert wird.

2. Verfahren gemäß Anspruch 1, wobei das flüssige Produkt eine wässrige Lösung ist.

3. Verfahren gemäß Anspruch 2, wobei die wässrige Lösung Proteine enthält.

4. Verfahren gemäß Anspruch 1, wobei die Teilchen des flüssigen Produkts einen Durchmesser von etwa 75 bis 590 µm haben.

5. Verfahren gemäß Anspruch 1, wobei die Teilchen des flüssigen Produkts sphärisch sind.

6. Verfahren gemäß Anspruch 1, wobei die kryogene Flüssigkeit angewendet wird, indem man die kryogene Flüssigkeit um die Tröpfchen des flüssigen Produkts herum umlaufend anordnet.

## Revendications

1. Procédé pour utiliser un liquide cryogénique destiné à produire des particules congelées d'un produit liquide dans un logement (6), le procédé comprenant :
(a) la production d'une paroi sensiblement continue annulaire dirigée vers le bas de liquide cryogénique (36) utilisant au moins trois buses de liquide cryogénique (12) pour définir une zone d'encapsulation intérieure (97) ;
(b) l'introduction des gouttelettes de produit liquide (63) dans une zone d'encapsulation (97) à l'aide d'une buse de produit liquide (8) positionnée dans le logement (6),
dans lequel le liquide cryogénique (36) congèle les gouttelettes de produit liquide (63) pour produire des particules congelées, **caractérisé par** le fait de
(c) on muni ledit logement d'un déflecteur (16) positionné au-dessus d'un tube de sortie (62) à l'intérieur de la buse de produit liquide (8) pour entourer une partie suffisante de la buse de produit liquide (8) afin de protéger la buse de produit liquide (8) des buses cryogéniques (12) pour ainsi empêcher la congélation du produit liquide à l'intérieur de la buse de produit liquide (8).

2. Procédé selon la revendication 1 dans lequel le produit liquide est une solution aqueuse.

3. Procédé selon la revendication 2 dans lequel la solution aqueuse contient des protéines.

4. Procédé selon la revendication 1 dans lequel les particules de produit liquide présentent un diamètre d'environ 75 à 590 microns.

5. Procédé selon la revendication 1, dans lequel les particules de produit liquide sont sphériques.

6. Procédé selon la revendication 1, dans lequel le liquide cryogénique est appliqué en disposant le liquide cryogénique de manière périphérique autour des gouttelettes des produits liquide.
